# FASCICULE DE BREVET EUROPEEN

(11) **EP 0 781 281 B1**
(45) Date de publication et mention de la délivrance du brevet: **19.12.2001**
(21) Numéro de dépôt: 95931243.0
(22) Date de dépôt: 14.09.1995
(51) Int. Cl.: C07D 471/04, C07D 487/04, C07D 221/10, C07D 471/14, A61K 31/435, A61K 31/55

(54) **DERIVES DE BETA-CARBOLINE AGONISTES DE LA MELATONINE, LEURS PROCEDES DE PREPARATION ET LEUR UTILISATION A TITRE DE MEDICAMENT**
BETA-CARBOLIN-DERIVATE ALS MELATONIN-AGONISTEN, IHRE HERSTELLUNGSVERFAHREN UND VERWENDUNG ALS ARZNEIMITTEL
BETA-CARBOLINE DERIVATIVES AS MELATONIN AGONISTS, METHODS FOR THEIR PREPARATION AND THEIR USE AS DRUGS

(30) Priorité: 14.09.1994 FR 9410964
(43) Date de publication de la demande: 02.07.1997
(73) Titulaire: MACEF, 33000 Bordeaux (FR); Laboratoires BESINS ISCOVESCO Société anonyme dite :, 75003 Paris (FR)
(72) Inventeur: FOURTILLAN, Jean-Bernard, F-86440 Migné-Auxances (FR); FOURTILLAN, Marianne, F-86440 Migné-Auxances (FR); JACQUESY, Jean-Claude, F-86180 Buxerolles (FR); JOUANNETAUD, Marie-Paule, F-86000 Poitiers (FR); VIOLEAU, Bruno, F-86370 Marcay (FR); KARAM, Omar Appartement 107, F-86000 Poitiers (FR)
(74) Mandataire: Ahner, Francis
(86) Numéro de dépôt international: FR9501179
(87) Numéro de publication internationale: WO9608490

(56) Documents cités:
- EP-A- 0 017 727
- EP-A- 0 466 548
- FR-A- 1 453 532
- FR-A- 1 524 953
- FR-A- 2 455 044
- FR-M- 1 825
- FR-M- 3 395
- US-A- 3 718 657
- US-A- 4 336 260
- US-A- 5 206 377
- CHEMICAL ABSTRACTS, vol. 109, no. 9, 1988 Columbus, Ohio, US; abstract no. 73711m, M. FERNADEZ 'Synthesis of 5,6,9,10,11,11a-hexahydrp-8h-naphtho[2,1-q uinolizines' page 721; & HETEROCYCLES, vol. 26, no. 12, 1987 pages 3059-3063,
- CHEMICAL ABSTRACTS, vol. 81, no. 17, 1974 Columbus, Ohio, US; abstract no. 105786m, S.V. KESSAR 'Aza steroids. ' page 561; & INDIAN J. CHEM. , vol. 12, no. 2, 1974 page 113-16
- CHEMICAL ABSTRACTS, vol. 98, no. 21, 1983 Columbus, Ohio, US; abstract no. 179750t, S.V. KESSAR 'Synthesis of 13-axaequilenin analogs from 1,2-dihydro-4-methylbenz[f]isoquinolines' page 695; & TETRAHEDRON LETT., vol. 23, no. 40, 1982 pages 4179-80,
- JOURNAL OF ORGANIC CHEMISTRY, vol. 26, 1961 EASTON US, pages 3086-3090, N.A. NELSON 'Steroidal hormone analogs'
- JOURNAL OF ORGANIC CHEMISTRY, vol. 39, no. 8, 1974 EASTON US, pages 1118-1124, N. FINCH ET AL. 'Synthesis of 1,3,4,5,6,7,8,8a-octahydro-2-methyl-4a-phe nylisoquinolin-6-ols.'
- JOURNAL OF THE AMERICAN CHEMICAL SOCIETY, vol. 98, no. 12, 1976 DC US, pages 6650-6657, D.D. WELLER ET AL. 'Synthesis of cis- and trans-4a-phenyldecahydroisoquinolines'
- JOURNAL OF HETEROCYCLIC CHEMISTRY, vol. 24, 1987 PROVO US, pages 623-628, Y. BENITO ET AL. 'Synthesis of 1'-methylspiro[3H-indole-3,n'-piperidines] from 1-methyl-n-piperidincaraldehydes'
- CHEMICAL ABSTRACTS, vol. 72, no. 21, 1970 Columbus, Ohio, US; abstract no. 111322n, R.G. GLUSHKOV ET AL. 'Opening the azepine ring in 2,3,4,5-tetrahydro-1H-azepino[3,4-b]indole during alkylation by alcohol in the presence of Raney nickel' page 390; & KHIM. GETEROTSIKL. SOEDIN. , 1970 pages 277-278,
- CHEMICAL ABSTRACTS, vol. 93, no. 25, 1980 Columbus, Ohio, US; abstract no. 239278d, B. A. SYDNEY ET AL. 'The reactions of some tetrahydro-beta-carbolines, of haxahydroazepino[3,4-b]indoles, and of tetrahydrocarbazolones with arenesulfonyl azides' page 841; & J. CHEM. SOC., PERKIN TRANS. 1, 1980 pages 1512-1515,
- CHEMICAL ABSTRACTS, vol. 111, no. 13, 1989 Columbus, Ohio, US; abstract no. 115071b, G.P. TOKMAKOV ET AL. 'Reactions of arylhydrazines with alpha-formyl derivatives of 6- and 7-membered lactams' page 653; & KHIM. GETEROTSIKL. SOEDIN, 1988 pages 1398-1403,
- JOURNAL OF ORGANIC CHEMISTRY, vol. 51, 1986 EASTON US, pages 3108-3112, A.I. MEEYERS ET AL. 'An asymetric synthesis and absolute configuration of (S)-(-)-deplancheine'
- TETRAHEDRON LETTERS, 1976 OXFORD GB, pages 1605-1608, SHUN-ICHI YAMADA ET AL 'A new entry into cinchona alkaloids via a biometic pathway'
- TETRAHEDRON LETTERS, vol. 33, no. 44, 1992 OXFORD GB, pages 6633-6636, D. GOMEZ-PRADO 'New approaches to corynanthe alkaloids involving the conjugate addition of dialkyl malonates to unsatuated thiolactams'
- JOURNAL OF ORGANIC CHEMISTRY, vol. 53, 1988 EASTON US, pages 4236-4241, S.B. MANDAL ET AL. 'Reduction of lactams and thiolactams by sodium borohydride: application in the synthesis of some alkaloids'
- ZEITSCHRIFT FUR NATURFORSCHUNG, TEIL B: ANORGANISCHE CHEMIE, ORGANISCHE CHEMIE, vol. 40B, 1985 TUBINGEN DE, pages 1747-1748, S. SIDDIQUI 'Reaction of harmidine/harmaline with BrCn'
- LIEBIGS ANNALEN DER CHEMIE, 1981 WEINHEIM DE, pages 1534-1544, H. MEYER 'Pyrrole duch cyclisierende Michael-Addition von Enaminen'
- JOURNAL OF THE CHEMICAL SOCIETY, PERKIN TRANSACTIONS 1, 1982 LETCHWORTH GB, pages 59-62, ATTA-UR-RAHMAN 'Synthesis of gambirtannine derivatives by photocyclization of enamine intermediates'
- BULLETIN DE LA SOCIETE CHIMIQUE DE FRANCE, vol. 129, 1992 PARIS FR, pages 303-307,
- JOURNAL OF ORGANIC CHEMISTRY, vol. 53, 1988 EASTON US, pages 2430-2434, A. BRANDI ET AL. 'Rearrangements of isoxazoline-5-spiro derivatives'
- EUROPEAN JOURNAL OF MEDICINAL CHEMISTRYCHIMICA THERAPEUTICA., vol. 21, no. 2, 1986 PARIS FR, pages 91-95,
- TETRAHEDRON, (INCL TETRAHEDRON REPORTS), vol. 47, no. 38, 1991 OXFORD GB, pages 8067-8078, Y. TORISAWA ET AL. 'A total synthesis of manzamine C and its geometrical isomer'
- J. Chem. Soc. Perkin Trans. I (1972), Vol.72(5), 736-738

## Description

La présente invention concerne de nouveaux dérivés de β-carboline, agonistes de la mélatonine, leur procédé de préparation et leur utilisation à titre de médicament.

La mélatonine, N-acétyl-5-méthoxytryptamine, est une hormone de la glande pinéale, isolée par Lerner & al. (J. Am. Chem. Soc., 80, 1958, 2587) et a fait l'objet de nombreuses études pour son activité circadienne, dans le rythme du sommeil, pour ses effets sur la production de testostérone, pour son activité au niveau de l'hypothalamus et dans les désordres psychiatriques.

Il a ainsi été envisagé d'employer la mélatonine et ses analogues, notamment pour le traitement de la dépression et des désordres psychiatriques, en particulier le stress, l'anxiété, la dépression, l'insomnie, la schizophrénie, les psychoses, l'épilepsie, mais également pour le traitement des troubles du sommeil liés aux voyages (« jet lag »), des maladies neurodégénératives du système nerveux central comme la maladie de Parkinson ou la maladie d'Alzheimer, pour le traitement de cancers, ou encore comme contraceptif, ou comme analgésique.

Toutefois, l'utilisation directe de la mélatonine *in vivo* ne s'est pas montrée très satisfaisante, compte tenu d'un premier passage hépatique qui extrait plus de 90% du principe actif.

Différents analogues de la mélatonine ont été décrits, mettant en évidence deux voies de recherche qui portent soit sur les substituants de la mélatonine (WO-A-89/01472, US-A-5 283 343, US-A-5 093 352 ou WO-A-93/11761) soit sur le noyau aromatique en remplaçant le groupe indolyle par un naphtyle (FR-A-2 658 818, FR-A-2 689 124).

La présente demande de brevet propose une nouvelle de voie de développement d'analogues de la mélatonine présentant une activité améliorée.
La présente invention concerne donc de nouveaux dérivés de carbolines de formule générale I 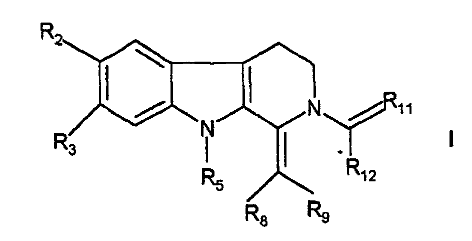 dans laquelle
- R₂ et R₃ représentent indépendamment l'un de l'autre un atome d'hydrogène, un radical hydroxyle, un radical alkyle en C₁-C₆, alkoxy dont le reste alkyle est en C₁-C₆, aryloxy, aralkoxy dont le reste alkyle est en C₁-C₆, halogéno, nitro
ou une chaîne hydrocarbonée insaturée en C₂-C₆, à condition que l'un au moins des substituants R₂ ou R₃ représente un radical hydroxyle ou alkoxy dont le reste alkyle est en C₁-C₆,
- R₅ représente un atome d'hydrogène ou un radical alkyle en C₁-C₆,
- R₈ et R₉ représentent, indépendamment l'un de l'autre, un atome d'hydrogène, un radical alkyle en C₁-C₆, un groupe phényle,
- R₁₂ représente un atome d'hydrogène, un radical alkyle en C₁-C₆, aryle, arakyle dont le reste alkyle est en C₁-C₆, une chaîne hydrocarbonée insaturée en C₂-C₆, chacun éventuellement substitué par un ou plusieurs halogènes, un radical amino, alkylamino dont le reste alkyle est en C₁-C₆, dialkylamino dont le reste alkyle est en C₁-C₆, arylamino, diarylamino, aralkylamino dont le reste alkyle est en C₁-C₆, ou aryl(alkyl)amino dont le reste alkyle est en C₁-C₆, arylcarbonyle, alkoxycarbonyle dont le reste alkyle est en C₁-C₆ ou un radical alkoxy dont le reste alkyle est en C₁-C₆,
- ou R₉ et R₁₂ sont liés pour former un cycle, comportant 5 ou 6 atomes, et pouvant être substitué par un ou plusieurs groupes représentant indépendamment l'un de l'autre un radical alkyle en C₁-C₆,
- R₁₁ représente un atome d'oxygène ou de soufre,
dans lesquels le terme aryle représente un groupe phényle, thiényle, furanyle, pyridyle ou naphtyle éventuellement substitué par un ou plusieurs substituants choisis parmi les radicaux alkyle en C₁-C₆, alkoxy dont le reste alkyle est en C₁-C₆ ou halogéno,
à l'exception de la 1-methylène-2-acétyl-7-méthoxy-1,2,3,4-tétrahydro-β-carboline,
leurs racémiques, leurs énantiomètres purs ou en mélange, et leurs sels thérapeutiquement acceptables.

Par alkyle, alkoxy ou perhalogénoalkyle, on entend de manière générale des radicaux dont le reste alkyle comprend entre 1 et 6 atomes de carbone.

Il s'agit de préférence de restes alkyles linéaires ou ramifiés en C₁-C₄, plus particulièrement choisis parmi les groupes méthyle, éthyle, n-propyle, i-propyle, n-butyle, i-butyle et t-butyle.

Par aryle, on désigne d'une manière générale les groupes aromatiques et hétéroaromatiques choisis parmi les groupes phényle, thiényle, furanyle, pyridyle ou naphtyte.

Les radicaux aryles peuvent également être substitués par un ou plusieurs substituants choisis parmi les radicaux alkyle, alkoxy ou halogéno définis ci-dessus.

Par aralkyle, on entendra la combinaison d'un alkyle et d'un aryle tels que définis ci-dessus. Il s'agira de préférence du radical benzyle, éventuellement substitué.

Les radicaux halogéno sont de préférence choisis parmi les atomes de fluor, de chlore, de brome ou d'iode.

De préférence, les radicaux perhalogénés sont des radicaux perfluorés.

Lorsque les dérivés comprennent au moins un carbone asymétrique, la présente invention concerne les racémiques correspondants, ainsi que ses énantiomères purs ou leurs mélanges en toutes proportions.

Les sels thérapeutiquement acceptables des dérivés selon l'invention sont les sels usuels de la technique, organiques ou inorganiques, notamment les chlorhydrates, les tosylates, les mésylates, les citrates ainsi que les solvates comme les hydrates ou hémihydrates des composés de formule générale I.

La présente invention concerne plus particulièrement les dérivés de formule génrale I, pour lesquels
- R₁₁ représente un atome d'oxygène.

De manière avantageuse, l'un au moins des substituants R₂ ou R₃ représente un radical méthoxy.

De manière préférentielle, R₁₂ représente un méthyle, un éthyle, un n-propyle, un aryle, un aralkyle dont le reste alkyle est en C₁-C₆, un radical perfluorométhyle, perfluoroéthyle, perfluoropropyle ou un radical arylamino.

Parmi les dérivés préférés selon l'invention, R₁₂ est avantageusement un radical alkyle, éventuellement lié à R₉ pour former un cycle, un méthyle, un éthyle, un n-propyle, un aryle, un aralkyle, un radical perfluorométhyle, perfluoroéthyle, perfluoropropyle ou un radical arylamino.

Différents dérivés de β-carboline ont été décrits dans l'état de la technique à titre d'intermédiaires de synthèse (M. Sainsbury *et al*., Journal of the Chemical Society, Perkin Transactions 1, vol.76, N°22, 1976, pages 2416-2418 ;Atta-Ur-Rahman, Joumal of the Chemincal Society, Perkin Transactions 1, vol.72, N°5, 1972, pages 736-738; T. Naito *et al*., Heterocycles, vol. 24, N°8, 1986 pages 2117-2120 ; R Grigg *et al*., Journal of the Chemical Society, Chemical Communications, vol.86, N°23, 1986, pages 1697-1699 ; F. Yamada *et al*., Heterocycles, vol. 24, N°9, 1986, pages 2619-2627 ; N. Takeaki *et al*., Heterocycles, vol. 26, N°7, 1987, pages 1739-1742 ; J. Laronze *et al*., Bulletin de la Société Chimique de France 2, Partie - Chimie Organique, Biochimie, vol.129, N°4, 1992, pages 303-307 ; R. Grigg *et al*., Tetrahedron, vol. 46, N°11, 1990, pages 4003-4018; G. Bobowski, Journal of Heterocyclic Chemistry, vol. 24, N°2, 1987, pages 473-479).

Il s'agit de dérivés de formule générale I pour lesquels
R₂ et R₅ représentent des atomes d'hydrogène,
R₃ représente un radical méthoxy,
R₄, substituant en position 8, représente un radical nitro,
R₈ et R₉ représentent des atomes d'hydrogène,
R₁₁ représente un atome d'oxygène et
R₁₂ représente un radical méthyle ou phényle.

Il s'agit également de dérivés de formule générale I pour lesquels
R₈ et R₉ représentent des atomes d'hydrogène,
R₂, R₃ et R₅ représentent des atomes d'hydrogène,
R₁₁ représente un atome d'oxygène,
R₁₂ représente un radical méthyle, trifluorométhyle, phényle, 2-iodo-phényle, pyridyle ou furyle; et de la 1-méthylène-2-acétyl-7-méthoxy-1,2,3,4-tétrahydro-β-carboline.

Les seuls dérivés de β-carboline décrits dans l'état de la technique présentant une activité thérapeutique sont les dérivés de l'harmaline décrits par Siddiqui et al. (Fitoterapia, vol. LXI, N°5, 1990, pages 425-433) qui présentent une activité antibactérienne.

Il s'agit de dérivés de formule générale I pour lesquels
R₈ et R₉ représentent des atomes d'hydrogène,
R₂, R₅ représentent des atomes d'hydrogène,
R₃ représente un radical méthoxy,
R₄ représente un radical nitro,
R₁₁ représente un atome d'oxygène et
R₁₂ représente un radical méthyle ou phényle.

De tels dérivés ne sont décrits que comme agent antibactérien.

La présente invention concerne également le procédé de préparation des dérivés de formule générale I, tels que définis ci-dessus.

Dans le cas particulier où R₁₁ représente un atome d'oxygène et R₉ et R₁₂ ne forment pas un cycle,
les dérivés de formule I peuvent être obtenus directement en faisant réagir les composés de formule générale IIa, 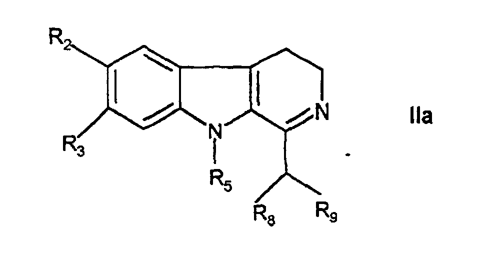 pour laquelle R₂, R₃, R₅, R₈ et R₉ sont définis ci-dessus, avec un agent acylant (chlorure d'acide, anhydride d'acide, chloroformiate, isocyanate) ou par échange avec un ester, on obtient alors les dérivés de formule l'a 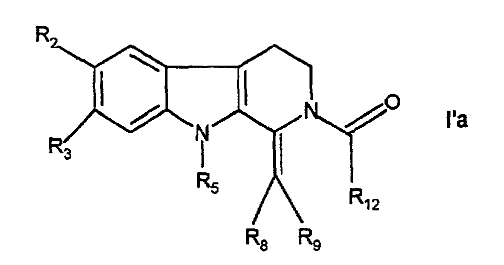 dans laquelle R₂, R₃, R₅, R₈, R₉, R₁₂ sont définis ci-dessus.

Pour obtenir les dérivés de formule générale IIa, on effectue une réaction de Bischler-Napieralski en faisant réagir les composés de formule générale IIIa 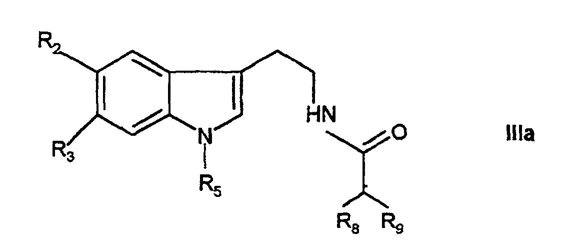 dans laquelle R₂, R₃, R₅, R₈ et R₉ sont définis ci-dessus, avec l'anhydride phosphorique (P₂O₅) ou l'oxychlorure de phosphore (POCl₃), dans un solvant approprié, par exemple le toluène ou le xylène.

Les dérivés de formule générale I, pour lesquels R₉ et R₁₂ sont liés pour former un cycle sont obtenus en effectuant une réaction de Bischler-Napieralski, dans les conditions décrites ci-dessus, sur une imide cyclique de formule générale III c, 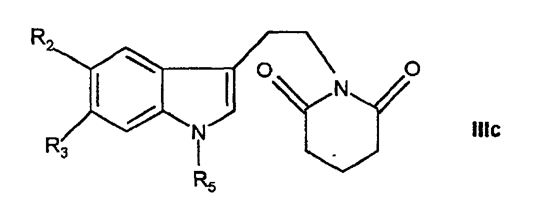 pour laquelle R₂, R₃, R₅ sont définis ci-dessus, on obtient alors le dérivé de formule générale l'c, 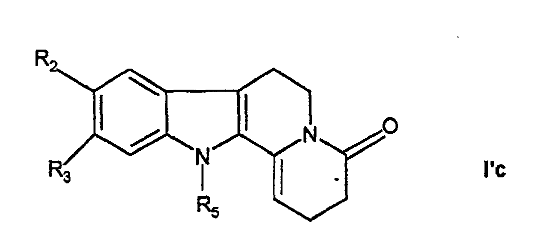 pour laquelle R₂, R₃, R₅ sont définis ci-dessus.

Les produits de départ de formule IIa comme le 10-méthoxyharmalan, et de formule III comme la mélatonine, la 5-méthoxytryptamine et la 4-méthoxyphénylhydrazine sont disponibles dans le commerce, ou peuvent être préparés à partir de ces dérivés.

La présente invention concerne également les dérivés de formules IIIa et IIIc, ainsi que les intermédiaires de formule IIa utiles en particulier pour la préparation de dérivés de formule générale I.

Les énantiomères des dérivés de formule I et leurs mélanges en toutes proportions pourront être obtenus par les méthodes usuelles de résolution de racémiques, notamment par cristallisation sélective en présence d'un acide.

Les exemples ci-après de préparations de dérivés selon l'invention permettent d'illustrer la présente invention sans toutefois chercher à en limiter la portée.

Des exemples de dérivés de formule l'a, pour lesquels R8 et R9 sont des atomes d'hydrogène, R₁₁ représente un atome d'oxygène, sont reportés sur le tableau I ci-après :

**TABLEAU I**

| **Exemple** | **R**_{**2**} | **R**_{**3**} | **R**_{**5**} | **R**_{**12**} | **Code** |
|---|---|---|---|---|---|
| **1** | OCH₃ | H | H | C₂F₅ | CARBO3 |
| **2** | OCH₃ | H | H | CF₃ | CARBO4 |
| **3** | OCH₃ | H | H | H | CARBO16 |
| **4** | OCH₃ | H | H | CH₃ | CARBO2 |
| **5** | OCH₃ | H | H | CH₂CH₃ | CARB02A |
| **6** | OCH₃ | H | H | CH₂CH₂CH₃ | CARBO2B |
| **8** | OCH₃ | H | H | Phényl | CARBO15 |
| **9** | OCH₃ | H | H | OCH₂CH₃ | CARBO5 |
| **10** | OCH₃ | H | H | NH-C₆H₅ | CARBO8 |
| **11** | OCH₃ | H | CH₃ | CH₃ | IMCARBO2 |
| **12** | OCH₃ | OCH₃ | H | CH₃ | DMCARBO2 |
| **13** | H | OCH₃ | H | CH₃ | CARBO1 |

Un exemple de dérivé de formule l'c, pour lequel R₂ représente un radical méthoxy, R₃ et R₅ représentent des atomes d'hydrogène, est défini à l'exemple 14 (code CARBO7).

La synthèse des produits de formules générales I, reportés ci-dessus, est présentée d'une manière détaillée ci-après.

### EXEMPLE 1

### 1-méthylène-2-pentafluoropropionyl-6-méthoxy-1,2,3,4-tétrahydro-β-carboline Formule : C₁₆H₁₃N₂O₂F₅ M = 360,28 g.mol⁻¹

A une solution de 10-méthoxyharmalan (150 mg) dans la pyridine (2 ml) on ajoute le PFPA (1,1 éq.). Après 5 min. de réaction on hydrolyse et on extrait par CH₂Cl₂. Le brut est ensuite flash-chromatographié (Eluant Et. de Pet / AcOEt ; 50/50). On récupère la 1-méthylène-2-pentafluoro-propionyl-6-méthoxy-1,2,3,4-tétrahydro-β-carboline (85 mg,rdt 35%).
Point de fusion 148-50°C.
RMN : 1H (CD₃COCD₃): 2,98 (t 6Hz, 2H, H-4) ;3,80 (s, 3H, OCH₃) ;4,18 (t 6Hz, H3) ; 5,39 (s large, 1H, H vin.) ; 5,71 (s, 1H, vin.) ; 6,83 (dd 8,8 et 3Hz, H-8) ; 7,01 (d 3Hz, 1H, H-5) ; 7,26 (d 8,8Hz, 1H, H-7) ; 8,47 (s large, 1H, N-H).
   ¹³C (CDCl₃) 22.3 (C-4) ; 46,4 (t 3Hz, C-3) ; 56,0 (OCH₃) ; 101,1 et 103,3 (C vin. et C-5) ; 112,1 et 114,1 (C-7 et C-8) ; 112,2 (C-4a) ; 127,1 et 132,3 (C-5a et C-8a) ; 129,8 (C-1a); 134,7 (C-1) ; 154,8 (C-6).
Spectre de masse : m/z : 360 (M⁺-), 241, 213
Masse exacte :
   calculée : 360,0897
   trouvée : 360,0888

### EXEMPLE 2

### 1-méthylène-2-trifluoroacétyl-6-méthoxy-1,2,3,4-tétrahydro-β-carboline Formule : C₁₅H₁₃N₂O₂F₃M = 310,28 g.mol⁻¹

A une solution de 10-méthoxyharmalan (40 mg) dans la triéthylamine (1 ml) on ajoute le TFA (2,2 éq.). Après 15 min. de réaction à 0*C, on hydrolyse et on extrait par CH₂Cl₂. Le brut est ensuite chromatographié sur plaque préparative (Eluant CH₂Cl₂). On recupère la 1-méthylène-2-trifluoroacétyl-6-méthoxy-1,2,3,4-tétrahydro-β-carboline (rdt 20%).
Point de fusion 181°C.
RMN : 1H (CD₃COCD₃) : 2,97 (t 5, 1Hz, 2H, H-4) ; 3,80 (s, 3H, OCH₃) ; 4,14 (t 5,1Hz, H-3) ; 5,37 (s large, 1H, H vin.) ; 5,70 (s, 1H, H vin.); 6,84 (dd 9,5 et 2,9Hz, H-8) ; 7,01 (d 2,9Hz, 1H, H-5) ; 7.26 (d 9,5Hz, 1H, H-7); 10,41 (s large, 1H, N-H).
   ¹³C (CDCl₃) : 22,1 (C-4) ; 46,4 (C-3) ; 56,0 (OCH₃) ; 101,1 et 103,6 (C vin. et C-5); 112,1 et 114,5 (C-7 et C-8) ; 112,2 (C-4a); 127,2 et 132,2 (C-5a et C-8a) ; 129,8 (C-1a) ; 136,4 (C-1) ; 154,8 (C-6).
Spectre de masse : m/z : 310 (M⁺·), 213, 185
Masse exacte :
   calculée: 310,0929
   trouvée : 310,0924

### EXEMPLE 3

### 1-méthylène-2-formyl-6-méthoxy-1,2,3,4-tétrahydro-β-carboline Formule : C₁₄H₁₄N₂O₂M = 242,27 g.mol⁻¹

A l'anhydride acétique (1 ml), refroidi à 0°C, on ajoute goutte à goutte l'acide formique (0,5 ml). L'ensemble est chauffé 15 min. à 50°C. Après refroidissement rapide à 0°C, on additionne lentement et par petites portions le 10-méthoxyharmalan, (200 mg), le milieu est laissé agiter 2h. Le brut est ensuite porté à sec et après séparation par chromatographie (Eluant AcOEt / Et. de Pet. ; 50/50) on obtient la 1-méthylène-2-formyl-6-méthoxy-1,2,3,4-tétrahydro-β-carboline.
Point de fusion 180-1°C.
RMN : 1H (CDCl₃) : 2,89 (t, 2H, H-4) ; 3,84 (s, 3H, OCH₃) ; 4,06 (t, H-3) ; 4,84 et 4,89 (2s, 2H, H vin.) ; 6.91 (d, 1H, H-7) ; 6,91 (s, 1H, H-5) ; 7,21 (d, 1H, H-8) ; 8,27 (s large, 1H, N-H), 8,7 (s, 1H, H formyl).
Spectre de masse: m/z: 242 ( M⁺-), 213 (100), 199, 170.

### EXEMPLE 4

### 1-méthylène-2-acétyl-6-méthoxy-1,2,3,4-tétrahydro-β-carboline Formule : C₁₅H₁₆N₂O₂M = 256.30 g.mol⁻¹

A une solution de 10-méthoxyharmalan (1 mmol) dans la pyridine (2 ml) on ajoute l'anhydride acétique (1,1 éq). Après hydrolyse acide et extraction à l'acétate d'éthyle, le brut est flash-chromatographié (Eluant AcOEt / Et. de Pet. ; 50/50). La 1-méthylène-2-acétyl-6-méthoxy-1,2,3,4-tétrahydro-β-carboline élue en premier.
Point de fusion 196-8°C.
RMN : ¹H(CDCl₃) : 2,29 (s, 3H, NCOCH₃); 2,85 (t 6Hz, 2H, H-4) ; 3,85 (s, 3H, OCH₃) ; 4,12 (t 6Hz, H-3) ; 4.97 (s large, 1H, H vin.); 5,35 (s, 1H, H vin.) ; 6,89 (dd 8 et 2Hz, H-7) ; 6,91 (s, 1H, H-5) ; 7,22 (d 8,6Hz, 1H, H-8) ; 8.47 (s large, 1H, N-H).
   ¹³C (CDCl₃):17,6 et 22,9 (C-4 et CH₃ amide) ; 43,6 (C-3); 56,0 (OCH₃) ; 101.1 et 101,3 (C vin. et C-5) : 111,9 et 114,1 (C-7 et C-8) ; 113,9 (C-4a) ; 127,3 et 132,3 (C-5a et C-8a) ; 130,5 (C-1a); 138,1 (C-1); 154,6 (C-6) ; 170,2 (C=O).
Spectre de masse : m/z : 256 ( M⁺-), 713 (M-COCH₃), 185, 170.
Masse exacte :
   calculée : 256,1212
   trouvée : 256,1208

### EXEMPLE 5

### 1-méthylène-2-propionyl-6-méthoxy-1,2,3,4-tétrahydro-β-carboline Formule : C₁₆H₁₈N₂O₂M = 270,33 g.mol⁻¹

A une solution de 10-méthoxyharmalan dans la pyridine on ajoute l'anhydride propionique. Après hydrolyse acide et extraction à l'acétate d'éthyle, le brut est ensuite flash-chromatographié (Eluant AcOEr / Et. de Pet. ; 50/50). La 1-méthylène-2-propionyl-6-méthoxy-1,2,3,4-tétrahydro-β-carboline élue en premier.
Point de fusion 174-75°C.
RMN : 1H (CDCl₃) : 1,96 (t, 3H, CH₃ éthyle) ; 2,63 (q, 2H, CH₂ éthyle) ; 2,83 (t, 2H, H-4) ; 3,85 (s, 3H, OCH₃) ; 4,12 (t, 2H, H-3) ; 5,01 (s large, 1H, H vin.) ; 5,37 (s, 1H, H vin.) ; 6,88 (dd, H-7) ; 6,91 (s, 1H, H-5) ; 7,22 (d, 1H, H-8) ; 8,53 (s large, 1H, N-H).
   ¹³C (CDCl₃):10,1 (CH₃ ethyle) ; 71,7 (C-4) ; 27,6 (CH₂ éthyle) ; 43,9 (C-3) ; 56,0 (OCH₃) ; 100,9 et 101,0 (C vin. et C-5) ; 112,0 et 114,1 (C-7 et C-8) ; 113,0 (C-4a) ; 127,0, 130,0 et 132,0 (C-5a, C-8a, C-1a) ; 137,0 (C-1) ; 154,5 (C-6) ; 174,0(C=O).
Spectre de masse : m/z : 270 (M⁺-), 213 (100), 170.
Masse exacte :
   calculée : 270,1368
   trouvée : 270,1364

### EXEMPLE 6

### 1-méthylène-2-butyryl-6-méthoxy-1,2,3,4-tétrahydro-β-carboline Formule : C₁₇H₂₀N₂O₂M = 284,35 g.mol⁻¹

A une solution de 10-méthoxyharmalan (1 mmol) dans la pyridine (2 ml) on ajoute l'anhydride butyrique (1,1 éq.). Après hydrolyse acide et extraction à l'acétate d'éthyle, le brut est ensuite flash-chromatographié (Eluant AcOEt / Et. de Pet. ; 50/50). La 1-méthylène-2-butyryl-6-méthoxy-1,2,3,4-tétrahydro-β-carboline élue en premier.
Point de fusion 188-190°C.
RMN : ¹H (CDCl₃) :0,91 (t, 3H, CH₃ propyle) ; 1,69 (m, 2H, CH₂ éthyle) ; 2,58 (m, 2H, CH₂-CO) : 2,83 (t, 2H, H-4) ; 3,85 (s, 3H, OCH₃) ; 4,13 (t, 2H, H-3) ; 5,01 (s large, 1H, H vin.) ; 5,35 (s, 1H, H vin.) ; 6,88 (dd, H-7) ; 6,91 (s, 1H, H-5) ; 7,23 (d, 1H, H-8) ; 8,46 (s large, 1H, N-H).
   ¹³C (CDCl₃) : 13.8 (CH₃ propyle) ; 19.3 (CH₂CH₃) ; 21.8 (C-4) ; 36.2 (CH₂CON) ; 43,9 (C-3) ; 55,9 (OCH₃) ; 101 et 101,6 (C vin. et C-5) ; 112 et 114 (C-7 et C-8) ; 113,5 (C-4a) ; 127,3, 130,7 et 132,2 (C-5a, C-8a, C-1a) ; 137,8 (C-1) : 154,5 (C-6) ; 173,2 (C=O).
Spectre de masse : m/z : 284 ( M⁺-), 270, 213 (100), 170.
Masse exacte :
   calculée : 284,1525
   trouvée : 284,1522

### EXEMPLE 8

### 1-méthylène-2-benzoyl-6-méthoxy-1,2,3,4-tétrahydro-β-carboline Formule : C₂₀H₁₈N₂O₂M = 318,37 g.mol⁻¹

A une solution de 10-méthoxyharmalan dans la pyridine on ajoute l'anhydride benzoïque. Après hydrolyse acide et extraction acétate d'éthyle, le brut est ensuite flash-chromatographié (Eluant AcOEt / Et de Pet ; 50/50). La 1-méthylène-2-benzoyl-6-méthoxy-1,2,3,4-tétrahydro-β-carboline élue en premier.
Point de fusion 192-3°C.
RMN : 1H (CDCl₃) : 2,95 (t 6Hz, 2H, H-4) ; 3,86 (s, 3H, OCH₃) ;4,20 (t 6Hz, H3) ; 4,43 (s large, 1H, H vin.) ; 5,06 (s, 1H, H vin.) ; 6,87 (dd,H-7) ; 6,95 (d,1H, H-5) ; 7,15 (d,1H, H-8) ; 7,30-7,45 (m,5H, H aromatiques de COC₆H₅) ; 8,33 (s large, 1H, N-H).
Spectre de masse : m/z :318 (M+), 289 (100), 213 (M-COC₆H₅) 170.
Masse exacte :
   calculée :318,1368
   trouvée : 318,1370

### EXEMPLE 9

### 1-méthylène-2-éthoxycarbonyl-6-méthoxy-1,2,3,4-tétrahydro-β-carboline formule : C₁₆H₁₈N₂O₃M = 286,33 g.mol-1

A une solution de 10-méthoxyharmalan dans le dichlorométhane on ajoute la triéthylamine (1,5 éq.), l'ensemble est refroidi à 0°C. On ajoute alors, goutte à goutte, une solution (1/5 v/v) de chloroformiate d'éthyle dans le dichlorométhane. L'ensemble est agité à 0°C jusqu'à disparition du produit de départ (2h). Après évaporation du solvant le brut est séparé sur plaque chromatographique (éluant AcOEt / Et. de Pet, 50/50).
Point de fusion 130-4°C.
RMN : 1H (CDCl₃) : 1,27 (t 3H, CH₃ de l'éthyle) ; 2,81 (t, 2H, H-4) ; 3,80 (s 3H, OCH₃) ; 4,01 (t, 2H, H-3) ; 4,20 (q, 2H, H-3) ; 5,34 (s, 1H, H vin.) ; 5,48 (s, 1H, H vin.) ; 6,79 (dd, H-7) ; 6,99 (d, 1H, H-5) ; 7,22 (d,1H, H-8) ; 10,22 (s large, 1H, N-H).

### EXEMPLE 10

### 1-méthylène-2-(N-phényl)carbamoyl-6-méthoxy-1,2,3,4-tétrahydro-β-carboline Formule : C₂₀H₁₉N₃O₂M = 333,38 g.mol-1

A une solution de 10-méthoxyharmalan dans l'éther anhydre on ajoute le phénylisocyanate. L'ensemble est agité pendant 1h30, puis chauffé 30 min. à 45°C. La méthylène-2-(N-phényl)carbamoyl-6-méthoxy-1,2,3,4-tétrahydro-β-carboline est récupérée par filtration puis lavée à l'éther.
Point de fusion 151-4°C.
RMN : 1H (DMSO/CDCl₃, 2/1) : 2,80 (m, 2H, H-6) ; 3,77 (s, 3H, OCH₃) ; 3,96 (m, H-7) ; 5,09 et 5,61 (2s, 2H, H vin.) ; 6,86 (m, 1H, H-7) ; 7,29 (d, 1H, H-5) ; 7,48 (d, 1H, H-8) ; 8,48 et 11,09 (2s large, 2H, 2N-H).
Spectre de masse : m/z :213 (M-120 CONHC₆H₅), 170, 119.

### EXEMPLE 11

### 1-méthylène-2-acétyl-6-méthoxy-9-méthyl-1,2,3,4-tétrahydro-p-carboline Formule : C₁₆H₁₈N₂O₂M = 270,33 g.mol⁻¹

Dans un ballon de 25 ml on dissout la mélatonine (600 mg) dans du DMSO (2 ml), on ajoute ensuite de la potasse (6 pastilles~300 mg). Après 15 min. d'agitation on additionne l'iodure de méthyle (0,6 ml). L'ensemble est agité une nuit, dilué à l'eau, puis acidifié avec de l'acide chlorhydrique 2N. Après extraction (dichlorométhane 3 fois), lavage à l'eau acide, séchage sur sulfate de magnésium et évaporation du solvant, on obtient la N-2-[(5-méthoxy-1-méthyl)indol-3-yl]éthyl acétamide FAB m/z 334 (MH⁺).

On effectue sur cette dernière une réaction de Bischler-Napieralski, avec du POCl₃ dans le toluène, pour obtenir le 1-méthyl-10-méthoxyharmalan.

A une solution de 1-méthyl-10-méthoxyharmalan dans la pyridine on ajoute l'anhydride acétique. Après hydrolyse acide et extraction à l'acétate d'éthyle, le brut est flash-chromatographié (Eluant AcOEt / Et. de Pet. ; 50/50). La 1-méthylène-2-acétyl-6-méthoxy-9-méthyl-1,2,3,4-tétrahydro-β-carboline élue en premier.
Point de fusion 142-3°C.
RMN : 1H (CDCl₃): 2,14 (s, 3H, NCOCH₃) ; 2,79 (t, 2H, H-4) ; 3,80 (s, 3H, NCH₃); 3,88 (s, 3H, OCH₃); 4,02 (t, 6Hz, H-3) ; 5,27 (s, 1H, H vin.) ; 5,69 (s, 1H, H vin) ; 6,89 (dd, H-7) ; 6,99 (s, 1H, H-5) ; 7,34 (d 8,6Hz, 1H, H-8).
   ¹³C (CDCl₃) : 21,6 et 22,4 (C-4 et CH₃ amide) ; 32,4 (CH₃-N) ; 43,3 (C-3) ; 56,0(OCH₃) ; 101 et 107,0 (C vin. et C-5) ; 110,3 et 114,0 (C-7 et C-8) ; 113,5 (C-4a) ; 126 et 131,5 et 134,9 (C-5a, C-8a et C-1a) ; 138,9 (C-1) ; 155 (C-6) ; 169,7 (C= O).
Spectre de masse : m/z :270 (M⁺), 255, 227 (100), 213
Masse exacte :
   calculée : 270,1368
   trouvée : 270,1370

### EXEMPLE 12

### 1-méthylène-2-acétyl-6,7-diméthoxy-1,2,3,4-tétrahydro-β-carboline Formule : C₁₆H₁₈N₂O₃M = 286,33 g.mol-1

A une solution de 10,11-diméthoxyharmalan dans la pyridine on ajoute l'anhydride acétique. Après hydrolyse acide et extraction à l'acétate d'éthyle, le brut est le flash-chromatographié (Eluant AcOEt / Et. de Pet. ; 50/50). La 1-méthylène-2-acétyl-6,7-diméthoxy-1,2,3,4-tétrahydro-p-carboline élue en premier.
RMN : 1H (CDCl₃) : 2,27 (s, 3H, NCOCH₃); 2,82 (t, 6Hz, 2H, H-4) ; 3,92 (s, 6H, 2OCH₃) ; 4,11 (t, 6Hz, H-3) ; 4,90 (s large, 1H, H vin.) ; 5,20 (s, 1H, H vin.) ; 6,85 et 6,89 (2s, 2H, H-5 et H-8); 7,95 (s large, 1H, NH).
Spectre de masse : m/z : 286 (M⁺), 243, 229
Masse exacte :
   calculée : 286,1317
   trouvée : 286,1320

### EXEMPLE 13

### 1-méthylène-2-acétyl-7-méthoxy-1,2,3,4-tétrahydro-β-carboline Formule : C₁₅H₁₆N₂O₂M= 256,30 g.mol⁻¹

A une solution d'harmaline (200 mg) dans le dichlorométhane (30 mL) on ajoute le chlorure d'acétyle (1,2 éq.). Après 30 min. de réaction, on additionne la triéthylamine (1,5 éq.). Après 5 min., on hydrolyse et on extrait par CH₂Cl₂, Le brut est ensuite flash-chromatographié (Eluant AcOEt / Et. de Pet. ; 60/40). La 1-méthylène-2-acétyl-7-méthoxy-1,2,3,4-tétrahydro-p-carboline¹ élue en premier (Rdt 50%).
¹O. Fischer, *Ber*, 1897, 30, 2481
Point de fusion 197-8°C.
RMN : 1H (CDCl₃): 2,21 (s, 3H, NCOCH₃) ; 2,77 (t, 6Hz, 2H, H-4) ; 3,80 (s, 3H, OCH₃); 4,03 (t 6Hz, H-3) ; 5,03 (s, 1H, H vin.) ; 5,49 (s, 1H, H vin.) ; 6,70 (dd 8,6 et 2Hz, H-6) ; 6,87 (s large, 1H, H-8) ; 7,36 (d 8,6Hz, 1H, H-5) ; 10,25 (s large, 1H, NH).
   ¹³C (CD₃COCD₃) : 22,3 et 23,2 (C-4 et CH₃ amide) ; 44,4 (C-3) ; 56,0 (OCH₃); 95,3 (C-8); 101,1 (C vin.) ; 110,6 (C-6) ; 113,9 (C-4a) ; 120,5 (C-5) ; 122,4 (C-5a) ; 130,3 (C-1a) ; 139,4 et 139,6 (C-8a et C-1); 158,7 (C-7) : 170,1 (C= O).
Spectre de masse : m/z: 256 (M⁺), 213 (M-COCH₃), 186,170.

### EXEMPLE 14

### 9-méthoxy-2,3,4,6,7,12-hexahydroindolo- [2,3-a] quinolizin-4-one Formule: C₁₆H₁₆N₂O₂M = 268,31 g.mol⁻¹

On chauffe à 175°C, un mélange de 5-méthoxytryptamine (420 mg) et du glutarate de diéthyle (460 mg), pendant 18h. La séparation sur colonne (éluant AcEOt), foumit l'amide ester 2. Ce dernier est traité par une trace d'acide paratoluènesulfonique dans du xylène, l'eau formée est éliminée à l'aide d'un Dean Stark. Après 9h de refilux et séparation sur plaque de silice on obtient le N[2-(5-méthoxy)indol-3-yléthyl)] glutarimide.

A une solution de N[2-(5-méthoxy)indol-3-yléthyl)] glutarimide (100 mg) dans le xylène à reflux on ajoute l'anhydride phosphorique (1,5 g)sous agitation. Après 5 heures de reflux, le milieu réactionnel est filtré. Le solide est repris à l'eau, alcalinisé (KOH, 40%), puis extrait à l'acétate d'éthyle. Après élimination du solvant la 9-méthoxy-2,3,4,6,7,12-hexahydroindolo [2,3-a] quinolizin-4-one est purifiée sur plaque de silice. (Eluant AcOEt / Et de Pet. 50/50).
Point de fusion 212-4°C.
RMN : 1H (CDCl₃): 2,46 et 2,63 (2m, 1H et 3H, 2H-2 et 2H-3) ; 2,87 (m, 2H, H-6) ; 3,85 (s, 3H, OCH₃) ; 4,10 (m, H-7) ; 5,50 (t, 1H, H-1) ; 6,87 (m, 1H, H-10) ; 6,94 (s, 1H, H-8); 7,22 (d, 1H, H-11) ; 11,67 (s large, 1H, H-12).
Spectre de masse : m/z :268 (M⁺), 216,187,173,159 (100).
Masse exacte
   calculée : 268,1211
   trouvée : 268,1210

### ACTIVITE BIOLOGIQUE

Les effets hypnotiques et sédatifs des dérivés, selon l'invention. préparés ci-dessus (dont les résultats des tests sont indiqués dans le Tableau I ci-après), ont été comparés à ceux de trois produits de référence, le diazépam, le pentobarbital sodique, la mélatonine ainsi qu'à 2 composés psycho-stimulants à propriétés hallucinogènes : le 10-méthoxy harmalan et l'harmaline qui sont des 3,4-dihydro-β-carbolines, chez des poussins de souche chair label JA657, âgés de 10 à 14 jours. Les animaux sont soumis à des programmes d'éclairement altemé comportant 12h d'obscurité (20h à 8h) et 12h d'éclairement (8h à 20h). La température d'ambiance est de 25°C pendant la première semaine d'élevage des poussins et de 22°C à partir de la deuxième semaine. Pendant la joumée, l'édairement est assuré par une lampe halogène (300 W), placée à 30 cm au-dessus du plancher du vivarium. Pendant les tests, les poids vifs des poussins ont varié entre 85 et 120 g. Les tests sont réalisés entre 14 et 15h. Les poussins sont allotés par groupes de 3, dans des vivariums identiques de 30 cm x 50 cm x 30 cm. Les produits testés sont administrés par voie intramusculaire (IM) dans le muscle pectoral majeur, en solution hydro-éthanolique (mélange éthanol/eau distillée, 50/50,V/V), à raison de 0,2 ml de solution éthanolique pour 100 g de poids vif. Les doses administrées pour les produits testés (nouveaux composés de l'invention et substances de référence) varient de 0,5 µMoles à 5 µMoles pour 100 g de poids vif. Le placebo correspond à 0,2 ml du mélange éthanol/eau distillée (aa) pour 100 g de poids vif. L'éthanol étant utilisé comme solvant, son effet a été comparé préalablement à celui du soluté physiologique (soluté NaCI à 0,9 p.100) ou de l'eau distillée.

Les solutions hydro-éthanoliques des produits testés ont été préparées extemporanément par dilution successive d'une solution mère, obtenue à partir de 5 à 50 µM de produit exactement pesées, additionnées de 1 ml d'éthanol pur, agitées aux ultrasons, puis complétées à 2 ml avec 1 ml d'eau distillée pour préparation injectable. Dans le tableau VII sont présentés les résultats obtenus après administration IM de doses comprises entre 0,5 et 5 µMoles de produits testés, en solution dans 0,2 ml du mélange éthanol/eau distillée , pour 100 g de poids vif. Pour chaque poussin, le volume injecté est ajusté, en fonction du poids vif réel, à 0,2 ml pour 100 g de poids vif.

Les paramètres observés sont l'activité locomotrice et l'état de veille des poussins pendant 2h, soit l'équivalent des 6 cycles théoriques veille-sommeil du poussin de cet âge. Ils sont enregistrés par caméra vidéo pendant 90 minutes, les 30 premières minutes étant le temps d'adaptation au dispositif.

Cinq stades de vigilance ont été définis :
- stade 1 : veilie active ;
- stade 2 : animal couché, maintien de la tête avec tonicité, oeil ouvert ;
- stade 3 : sommeil léger, animal assoupi; oeil fermé avec ouverture intermittente, posture immobile non modifiée par la stimulation ;
- stade 4: sommeil profond couché: relâchement du cou, posture caractéristique tête sous l'aile ou en arrière ;
- stade 5 : sommeil debout :oeil fermé, immobile, tête tombante (catatonique).

Ces cinq stades correspondent approximativement aux stades de vigilance et de sommeil définis à l'examen des tracés électro-encéphalographiques dans cette espèce. La correspondance est la suivante :
■ Sommeil profond couché : stade 4 = « slow wave sleep » (SWS)
■ Sommeil debout = « sleep-like state I » (SLSI).

Le stade 3, assoupi, pourrait correspondre à des phases de sommeil paradoxal, avec agitation de la tête, par exemple.

L'observation des poussins est réalisée par un observateur entraîné avec un contrôle vidéo continu pendant au moins une heure après le réveil des animaux.

Deux stimuli ont été utilisés pour confirmer les observations du comportement des poussins à intervalles réguliers :
- le bruit causé par le choc d'un objet en plastique sur la vitre du vivarium, comparable à celui du bec d'un poussin sur la vitre, correspond à un stimulus modéré. Il est pratiqué à chaque période d'observation (soit toutes les 5 minutes) ;
- et la présentation d'une mangeoire métallique remplie avec l'aliment habituel, laissée 2 minutes dans le vivarium. Il s'agit d'un stimulus puissant faisant appel à la vision, l'ouïe et l'odorat. Elle est pratiquée toutes les 15 minutes, c'est à dire 6 fois, au moins, à chaque essai.

Le réveil est défini par l'apparition du comportement élaboré conscient de recherche et consommation de nourriture ou de boisson.

Le Temps de Sommeil (TS) et défini par la somme des durées des phases de sommeil léger (stade 3), sommeil profond (stade 4) et sommeil debout (stade 5). Le Temps de Sédation, postérieur au réveil, correspond au stade 2.

Le Temps d'Assoupissement (TA) est égal (à 1 minute près) au temps nécessaire au passage d'état de veille active (stade 1) à un état non vigile (stades 3, 4 et 5).

Les effets hypnotiques et sédatifs des produits d'essai, sur l'activité diume de poussins, âgés de 10 à 14 jours, soumis à un programme d'éclairement permanent dès la naissance pendant 48h, puis à un programme d'éclairement altemé de 12h de jour (8h - 20h) et 12h d'obscurité (20h - 8h) jusqu'au jour de l'essai, sont reportés au Tableau VII ci-après. Les tests sont réalisés le jour entre 14h et 15h.

Pour chaque produit testé, plusieurs séries de mesures ont été réalisées sur des lots de 3 animaux, chaque valeur indiquée est la moyenne dans chaque lot de 3 poussins. Lorsque le nombre de lots est supérieur ou égal à 2, les chiffres indiqués sont les valeurs moyennes limites observées.

**TABLEAU VII**

| COMPOSE | DOSE (µM/100g) | DOSE (mg/kg) | TA (min.) | TS (min.) | Temps de Sédation (min.) |
|---|---|---|---|---|---|
| Placebo | (20 lots) | | NA | 0 | 10-65 |
| Mélatonine | 0.5 | 1,16 | NA | 0 | Non déterminé |
| | 1 (5 lots) | 2.32 | NA | 0 | 16 - 36 |
| | 2 (5 lots) | 4,64 | NA | 0 | 47 - 105 |
| Pentobarbital | 0.5 (3 lots) | 1.24 | NA | 0 | Non déterminé |
| | 1 | 2,48 | 13 | 36 | Non déterminé |
| Diazépam | 0.5 (4 lots) | 1,42 | 3 - 6 | 10 - 50 | Non déterminé |
| | 1 (10 lots) | 2,85 | 2 - 7 | 24 - 70 | 17 - 20 |
| | 2 (3 lots) | 5.69 | 2 - 5 | 81-100 | 14 - 15 |
| 10-méthoxy harmalan | 1.4 | 3 | NA | 0 | 0 |
| Harmaline | 1,4 | 3 | NA | 0 | 0 |
| 1 | 0.5 | 1.80 | NA | 0 | 35 |
| | 1 | 3.60 | 9 | 29 | 1 |
| 2 | 0.5 | 1.55 | 10 | <7 | 30 |
| | 1 | 3.10 | 7 | 7 | 43 |
| 3 | 2 | 4.85 | 5-5 | 25-60 | 32-63 |
| 4 | 0.5 (3 lots) | 1.28 | 4-8 | 2-48 | 12-38 |
| | 1 (8 lots) | 2.56 | 2-9 | 36-65 | 3-4 |
| | 2 (10 lots) | 5.12 | 4-11 | 40-70 | 22-25 |
| | 5 (4 lots) | 12.80 | 2-7 | 58-90 | 13-25 |
| 5 | 1 | 2.70 | 3 - 5 | 40-57 | 17-31 |
| 6 | 1 | 2.84 | 4 - 6 | 67-85 | 2-18 |
| | | | | | |
| 8 | 2 | 6.37 | 10-10 | 20-30 | 65-70 |
| 9 | 1 | 2.86 | NA-10 | 0-<5 | 21-26 |
| 10 | 2 | 6.67 | 5-10 | 40-56 | 21-23 |
| 11 | 2 | 5.40 | 5-6 | 51-60 | 16-23 |
| 13 | 0,5 | 1.28 | NA | 0 | 8 |
| | 1 | 2.56 | 11 | 30 | 8 |
| 14 | 2 | 5,37 | 4-7 | 52-72 | 2-5 |
| Légendes : - NA : non applicables, les animaux restent vigiles pendant toute la période de l'observation ; - TA : temps d'assoupissement égal au temps nécessaire pour passer de l'état de veille active à un état non vigile ; - TS : temps de sommeil, égal à la durée de la période de sommeil allant de l'endormissement au réveil ; - Temps de Sédation : postérieur au réveil, période d'inactivité correspondant au stade 2 défini ci-dessus. | | | | | |

Dans les conditions de réalisation du test (horaires d'administration, pendant la phase d'éclairement des animaux, entre 14 h et 15 h) l'activité hypnotique de la mélatonine est nulle.

En soumettant successivement des poussins à des programmes d'éclairements alterné et permanent, nous avons démontré expérimentalement que la mélatonine n'a pas d'activité hypnotique directe, propre à sa structure. Son activité hypnotique dépend de l'activité de l'Enzyme N Acétyl Transférase (NAT) dans la glande pinéale du poussin au moment de l'administration de la mélatonine. L'enzyme NAT est une enzyme d'acétylation. En présence de l'enzyme NAT dans la glande pinéale du poussin, l'administration IM de mélatonine induit un effet hypnotique de forte intensité (temps de sommeil compris entre 250 et 300 minutes pour une dose égale à 1 µM de mélatonine / 100 g de poids vif). La mélatonine est donc le précurseur de métabolites acétylés à activité hypnotique directe, parmi lesquels figure le composé 4.

Contrairement à la mélatonine, tous les dérivés de l'invention décrits ci-dessus ont des activités hypnotiques et sédatives directes, qui sont indépendantes de l'heure d'administration, c'est-à-dire du taux de l'enzyme N-Acétyl Transférase dans le SNC.

Les résultats obtenus montrent, pour les dérivés selon l'invention, un effet hypnotique supérieur à celui des produits de référence (pentobarbital, mélatonine) et équivalent ou méme supérieur à celui du diazépam.

Les dérivés selon l'invention sont donc particulièrement avantageux pour le traitement de maladies liées aux désordres de l'activité de la mélatonine.

La présente invention concerne donc les dérivés de formule générale I, tels que définis précédemment pour leur utilisation en thérapie, notamment pour le traitement de la dépression et des désordres psychiatriques, en particulier le stress, l'anxiété, la dépression, l'insomnie, la schizophrénie, les psychoses, l'épilepsie, mais également pour le traitement des troubles du sommeil liés aux voyages ("jet lag"), des maladies neurodégénératives du système nerveux central comme la maladie de Parkinson ou la maladie d'Alzheimer, pour le traitement de cancers, ou encore comme contraceptif, ou comme analgésique.

Les analogues mélatoninergiques selon l'invention sont également utiles pour le traitement de l'hyperplasie bénigne de la prostate, des cancers de la peau, les affections de la peau comme le psoriasis, l'acné, les mycoses, du glaucome, ainsi que pour augmenter les résistances immunitaires.

Ils sont également utiles pour prévenir les symptômes de la ménopause, les syndrômes pré-menstruels, les effets du vieillissement et la mort subite du nouveau-né.

Ils sont également utiles en application vétérinaire pour réguler les naissances chez les animaux ruminants.

La présente invention concerne donc également les compositions pharmaceutiques adaptées pour l'administration des dérivés de formule générale I, notamment par voie orale, parentérale, rectale, sous la forme de capsules, comprimés, gélules, solutions buvables, solutions injectables, y compris les formes retard et les pansements à libération prolongée pour l'administration transdermique du principe actif, les spray nasals, ou les formulations topiques (crème, émulsion, etc.), comprenant un dérivé de formule générale I selon l'invention et au moins un excipient acceptable pharmaceutiquement.

Les compositions pharmaceutiques selon l'invention sont avantageusement dosées pour délivrer le principe actif en une seule "prise". Pour une administration par voie orale, les doses unitaires efficaces sont comprises entre 0,1 µg et 500 mg.

Pour une administration intraveineuse, les doses unitaires efficaces sont comprises entre 0,1 µg et 100 mg.

Les analogues mélatoninergiques selon l'invention sont également utiles en cosmétique, notamment pour la protection de la peau contre le vieillissement, mais également contre la chute des cheveux.

La présente invention concerne donc également une composition cosmétique comprenant un dérivé de formule générale I selon l'invention.

Les compositions cosmétiques selon l'invention sont formulées d'une manière appropriée, pour leur application topique, notamment sous la forme de pommades, crémes, émulsions, onguents, lotions, etc.

## Revendications

1. Dérivés de carbolines de formule générale I 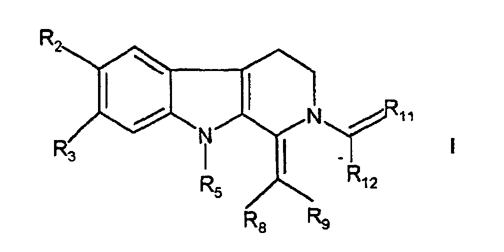 dans laquelle
- R₂ et R₃ représentent indépendamment l'un de l'autre un atome d'hydrogène, un radical hydroxyle, un radical alkyle en C₁-C₆, alkoxy dont le reste alkyle est en C₁-C₆, aryloxy, aralkoxy dont le reste alkyle est en C₁-C₆, halogéno, nitro ou une chaîne hydrocarbonée insaturée en C₂-C₆, à condition que l'un au moins des substituants R₂ ou R₃ représente un radical hydroxyle ou alkoxy dont le reste alkyle est en C₁-C₆,
- R₅ représente un atome d'hydrogène ou un radical alkyle en C₁-C₆,
- R₈ et R₉ représentent, indépendamment l'un de l'autre, un atome d'hydrogène, un radical alkyle en C₁-C₆, un groupe phényle,
- R₁₂ représente un atome d'hydrogène, un radical alkyle en C₁-C₆, aryle, arakyle dont le reste alkyle est en C₁-C₆, une chaîne hydrocarbonée insaturée en C₂-C₆, chacun éventuellement substitué par un ou plusieurs halogènes, un radical amino, alkylamino dont le reste alkyle est en C₁-C₆, dialkylamino dont le reste alkyle est en C₁-C₆, arylamino, diarylamino, aralkylamino dont le reste alkyle est en C₁-C₆, ou aryl(alkyl)amino dont le reste alkyle est en C₁-C₆, arylcarbonyle, alkoxycarbonyle dont le reste alkyle est en C₁-C₆ ou un radical alkoxy dont le reste alkyle est en C₁-C₆,
- ou R₉ et R₁₂ sont liés pour former un cycle, comportant 5 ou 6 atomes, et pouvant être substitué par un ou plusieurs groupes représentant indépendamment l'un de l'autre un radical alkyle en C₁-C₆,
- R₁₁ représente un atome d'oxygène ou de soufre,
dans lesquels le terme aryle représente un groupe phényle, thiényle, furanyle, pyridyle ou naphtyle éventuellement substitué par un ou plusieurs substituants choisis parmi les radicaux alkyle en C₁-C₆, alkoxy dont le reste alkyle est en C₁-C₆ ou halogéno,
à l'exception de la 1-méthylène-2-acétyl-7-méthoxy-1,2,3,4-tétrahydro-β-carboline,
leurs racémiques, leurs énantiomètres purs ou en mélange, et leurs sels thérapeutiquement acceptables.

2. Dérivés de formule générale I selon la revendication 1, **caractérisés en ce que** R₁₁ représente un atome d'oxygène.

3. Dérivés de formule générale I selon l'une des revendications 1 ou 2, **caractérisés en ce que** l'un au moins des substituants R₂ ou R₃ représente un radical méthoxy.

4. Dérivés de formule générale I selon l'une des revendications 1 à 3, **caractérisés en ce que** R₁₂ représente un méthyle, un éthyle, un n-propyle, un aryle, un arakyle dont le reste alkyle est en C₁-C₆, un radical perfluorométhyle, perfluoroéthyle, perfluoropropyle ou un radical arylamino.

5. Dérivés de formule générale I selon la revendication 1, **caractérisés en ce qu'**ils sont sélectionnés parmi les dérivés suivants :
1-méthylène-2-pentafluoropropionyl-6-méthoxy-1,2,3,4-tétrahydro-β-carboline
1-méthylène-2-trifluoroacétyl-6-méthoxy-1,2,3,4-tétrahydro-β-carboline
1-méthylène-2-formyl-6-méthoxy-1,2,3,4-tétrahydro-p-carboline
1-méthylène-2-acétyl-6-méthoxy-1,2,3,4- tétrahydro-β-carboline
1-méthylène-2-propionyl-6-méthoxy-1,2,3,4- tétrahydro-β-carboline
1-méthylène-2-butyryl-6-méthoxy-1,2,3,4-tétrahydro-β-carboline
1-méthylène-2-benzoyl-6- méthoxy-1,2,3,4- tétrahydro-β-carboline
1-méthylène-2-éthoxycarbonyl-6-méthoxy-1,2,3,4- tétrahydro-β-carboline
1-méthylène-2-(N-phényl)carbamoyl-6- méthoxy-1,2,3,4-tétrahydro-β-carboline
1-méthylène-2-acétyl-6-méthoxy-9-méthyl-1,2,3,4- tétrahydro-p-carboline
1-méthylène-2-acétyl-6,7-diméthoxy-1,2,3,4-tétrahydro-β-carboline
9-méthoxy-2,3,4,6,7,12-hexahydroindolo [2,3-a]quinolizin-4-one

6. Médicament, **caractérisé en ce qu'**il comprend au moins un dérivé de β-carboline de formule générale I 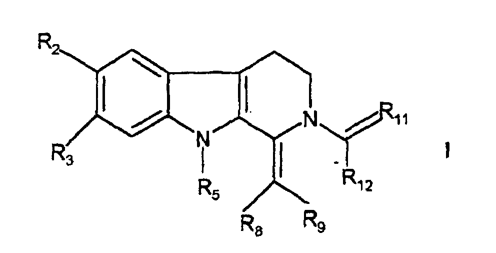 selon l'une des revendications 1 à 5 ou la 1-méthylène-2-acétyl-7-méthoxy-1,2,3,4-tétrahydro-β-carboline.

7. Médicament selon la revendication 6, **caractérisé en ce qu'**il s'agit d'un médicament hypnotique.

8. Médicament selon la revendication 6 ou 7 **caractérisé en ce qu'**il comprend un dérivé sélectionné parmi :
1-méthylène-2-pentafluoropropionyl-6-méthoxy-1,2,3,4-tétrahydro-β-carboline
1-méthylène-2-trifluoroacétyl-6-méthoxy-1,2,3,4-tétrahydro-β-carboline
1-méthylène-2-formyl-6-méthoxy-1,2,3,4-tétrahydro-β-carboline
1-méthylène-2-acétyl-6-méthoxy-1,2,3,4-tétrahydro-β-carboline
1-méthylène-2-propionyl-6-méthoxy-1,2,3,4- tétrahydro-p-carboline
1-méthylène-2-butyryl-6- méthoxy-1,2,3,4-tétrahydro-β-carboline
1-méthylène-2-benzoyl-6- méthoxy-1,2,3,4- tétrahydro-β-carboline
1-méthylène-2-éthoxycarbonyl-6-méthoxy-1,2,3,4- tétrahydro-β-carboline
1-méthylène-2-(N-phényl)carbamoyl-6-méthoxy-1,2,3,4-tétrahydro-β-carboline
1-méthylène-2-acétyl-6-méthoxy-9-méthyl-1,2,3,4- tétrahydro-β-carboline
1-méthylène-2-acétyl-6,7-diméthoxy-1,2,3,4-tétrahydro-β-carboline
1-méthylène-2-acétyl-7-méthoxy-1,2,3,4-tétrahydro-β-carboline
9-méthoxy-2,3,4,6,7,12-hexahydroindolo [2,3-a]quinolizin-4-one

9. Composition cosmétique, **caractérisée en ce qu'**elle comprend comme composé cosmétiquement actif, un dérivé de formule générale I, 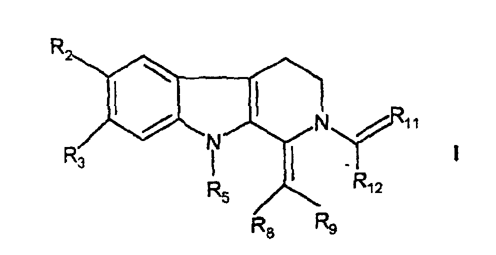 selon l'une des revendications 1 à 5.

10. Procédé de préparation des dérivés de formule générale I, 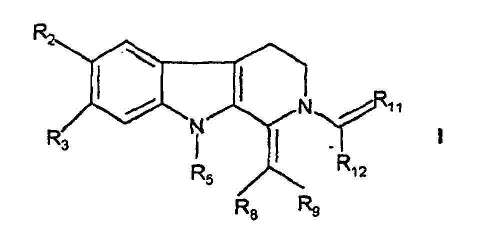 selon les revendications 1 à 5, **caractérisé en ce que** l'on met en oeuvre l'une des réactions suivantes :
**10.1.** lorsque R₁₁ représente un atome d'oxygène et que R₉ et R₁₂ ne forment pas un cycle, les dérivés de formule I peuvent être obtenus directement en faisant réagir les composés de formule générale IIa, 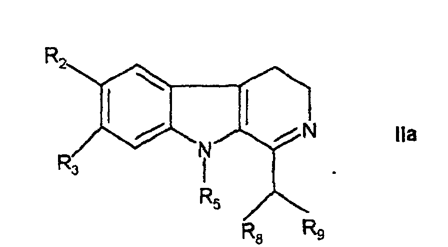 pour laquelle R₂,R₃,R₅,R₈,R₉, sont définis ci-dessus, avec un agent acylant (chlorure d'acide, anhydride d'acide, chtoroformiate, isocyanate) ou par échange avec un ester, on obtient alors les dérivés de formule l'a 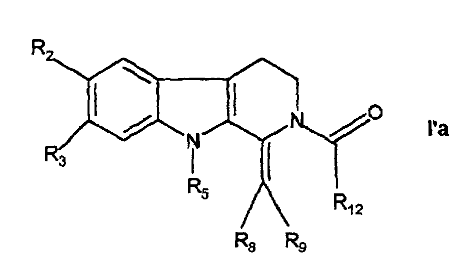 dans laquelle R₂,R₃,R₅,R₈,R₉, et R₁₂, sont définis ci-dessus ;
**10.2.** Pour obtenir les dérivés de formule générale IIa, on effectue une réaction de Bischler-Napieralski en faisant réagir les composés de formule générale IIIa 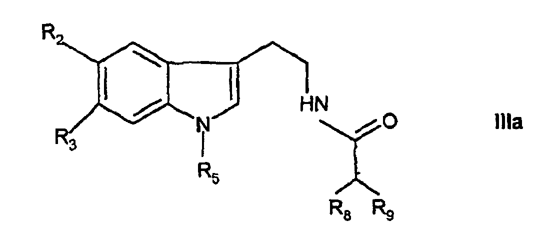 dans laquelle R₂, R₃, R₅, R₈, R₉, sont définis ci-dessus, avec l'anhydride phosphorique (P₂O₅) ou l'oxychlorure de phosphore (POCl₃), dans un solvant approprié, par exemple le toluène ou le xylène ;
**10.3.** Les dérivés de formule générale I, pour lesquels R₉ et R₁₂ sont liés pour former un cycle sont obtenus en effectuant une réaction de Bischler-Napieralski, dans les conditions décrites ci-dessus, sur une imide cyclique de formule générale IIIc, 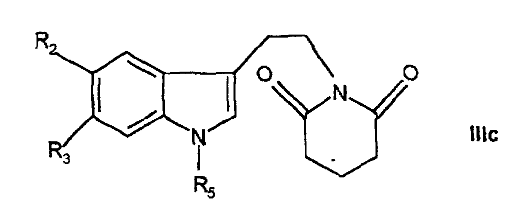 pour laquelle R₂, R₃, R₅ sont définis ci-dessus, on obtient alors le dérivé de formule générale l'c, 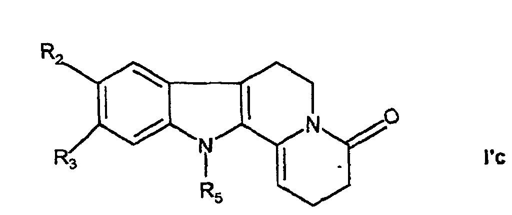 pour laquelle R₂, R₃, R₅ sont définis ci-dessus.

## Patentansprüche

1. Carbolinderivate der allgemeinen Formel I 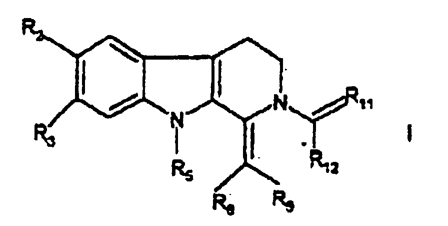 worin
- R₂ und R₃ unabhängig voneinander ein Wasserstoffatom, ein Hydroxylradikal, ein Alkylradikal mit C₁-C₆, Alkoxy mit einem Alkylrest von C₁-C₆, Aryloxy, Aralkoxy mit einem Alkylrest von C₁-C₆, Halogen, Nitro oder eine ungesättigte Kohlenwasserstoffkette mit C₂-C₆ darstellen, unter der Voraussetzung, dass wenigstens einer der Substituenten R₂ oder R₃ ein Hydroxylradikal oder ein Alkoxy mit einem Alkylrest von C₁-C₆ darstellt,
- R₅ ein Wasserstoffatom oder ein Alkylradikal mit C₁-C₆ darstellt,
- R₈ und R₉ unabhängig voneinander ein Wasserstoffatom, ein Alkylradikal mit C₁-C₆ oder eine Phenylgruppe darstellen,
- R₁₂ ein Wasserstoffatom, ein Alkylradikal mit C₁-C₆, Aryl, Arakyl mit einem Alkylrest von C₁-C₆, eine ungesättigte Kohlenwasserstoffkette mit C₂-C₆, jeweils gegebenenfalls substituiert durch ein oder mehrere Halogene, ein Aminoradikal, Alkylamino mit einem Alkylrest von C₁-C₆, Dialkylamino mit·einem Alkylrest von C₁-C₆, Arylamino, Diarylamino, Aralkylamino mit einem Alkylrest von C₁-C₆, oder Aryl(alkyl)amino mit einem Alkylrest von C₁-C₆, Arylcarbonyl, Alkoxycarbonyl mit einem Alkylrest von C₁-C₆ oder ein Alkoxyradikal mit einem Alkylrest von C₁-C₆, darstellt,
- oder R₉ und R₁₂ zur Bildung eines Zyklus verbunden sind, welcher 5 oder 6 Atome aufweist und durch eine oder mehrere Gruppen, die unabhängig voneinander ein Alkylradikal mit C₁-C₆ darstellen, substituiert sein kann,
- R₁₁ ein Sauerstoff- oder Schwefelatom darstellt,
wobei der Ausdruck Aryl eine Phenyl-, Thienyl-, Furanyl-, Pyridyl- oder Naphtylgruppe, gegebenenfalls substituiert durch ein oder mehrere der Substituenten Alkylradikale mit C₁-C₆, Alkoxy mit einem Alkylrest von C₁-C₆ oder Halogen, darstellt,
ausgenommen, das 1-Methylen-2-acetyl-7-methoxy-1,2,3,4-tetrahydro-β-carbolin,
ihre Racemate, ihre reinen Enantiomeren oder deren Mischungen und ihre therapeutisch geeigneten Salze.

2. Derivate der allgemeinen Formel I gemäß Anspruch 1, **dadurch gekennzeichnet, dass** R₁₁ ein Sauerstoffatom darstellt.

3. Derivate der allgemeinen Formel I gemäß einem der Ansprüche 1 oder 2, **dadurch gekennzeichnet, dass** wenigstens einer der Substituenten R₂ oder R₃ ein Methoxyradikal darstellt.

4. Derivate der allgemeinen Formel I gemäß einem der Ansprüche 1 bis 3, **dadurch gekennzeichnet, dass** R₁₂ ein Methyl, ein Ethyl, ein n-Propyl, ein Aryl, ein Arakyl mit einem Alkylrest von C₁-C₆, ein Perfluormethyl-, Perfluorethyl-, Perfluorpropyl- oder Arylaminoradikal darstellt.

5. Derivate der allgemeinen Formel I gemäß Anspruch 1, **dadurch gekennzeichnet, dass** sie aus folgenden Derivaten ausgewählt sind:
l-Methylen-2-pentafluorpropionyl-6-methoxy-1,2,3,4-tetrahydro-β-carbolin
1-Methylen-2-trifluoracetyl-6-methoxy-1,2,3,4-tetrahydro-ß-carbolin
l-Methylen-2-formyl-6-methoxy-1,2,3,4-tetrahydro-ß-carbolin
1-Methylen-2-acetyl-6-methoxy-1,2,3,4-tetrahydro-ß-carbolin
l-Methylen-2-propionyl-6-methoxy-1,2,3,4-tetrahydro-β-carbolin
l-Methylen-2-butyryl-6-methoxy-1,2,3,4-tetrahydro-ß-carbolin
1-Methylen-2-benzoyl-6-methoxy-1,2,3,4-tetrahydro-ß-carbolin
1-Methylen-2-ethoxycarbonyl-6-methoxy-1,2,3,4-tetrahydro-β-carbolin
l-Methylen-2-(N-phenyl)carbamoyl-6-methoxy-1,2,3,4-tetrahydro-β-carbolin
1-Methylen-2-acetyl-6-methoxy-9-methyl-1,2,3,4-tetrahydro-β-carbolin
l-Methylen-2-acetyl-6,7-dimethoxy-1,2,3,4-tetrahydro-ß-carbolin
9-Methoxy-2,3,4,6,7,12-hexahydroindolo[2,3-a]quinolizin-4-on.

6. Medikament, **dadurch gekennzeichnet, dass** es wenigstens ein β-Carbolinderivat der allgemeinen Formel I 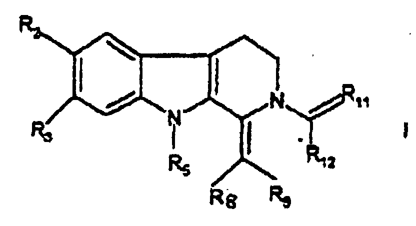 gemäß einem der Ansprüche 1 bis 5 oder 1-Methylen-2-acetyl-7-methoxy-1,2,3,4-tetrahydro-β-carbolin enthält.

7. Medikament gemäß Anspruch 6, **dadurch gekennzeichnet, dass** es sich um ein hypnotisches Medikament handelt.

8. Medikament gemäß Anspruch 6 oder 7, **dadurch gekennzeichnet, dass** es wenigstens eines der folgenden Derivate enthält:
1-Methylen-2-pentafluorpropionyl-6-methoxy-1,2,3,4-tetrahydro-β-carbolin
l-Methylen-2-trifluoracetyl-6-methoxy-1,2,3,4-tetrahydro-β-carbolin
1-Methylen-2-formyl-6-methoxy-1,2,3,4-tetrahydro-β-carbolin
1-Methylen-2-acetyl-6-methoxy-1,2,3,4-tetrahydro-β-carbolin
1-Methylen-2-propionyl-6-methoxy-1,2,3,4-tetrahydro-ß-carbolin
1-Methylen-2-butyryl-6-methoxy-1,2,3,4-tetrahydro-β-carbolin
1-Methylen-2-benzoyl-6-methoxy-1,2,3,4-tetrahydro-ß-carbolin
1-Methylen-2-ethoxycarbonyl-6-methoxy-1,2,3,4-tetrahydro-ß-carbolin
1-Methylen-2-(N-phenyl)carbamoyl-6-methoxy-1,2,3,4-tetrahydro-β-carbolin
l-Methylen-2-acetyl-6-methoxy-9-methyl-1,2,3,4-tetrahydro-β-carbolin
l-Methylen-2-acetyl-6,7-dimethoxy-1,2,3,4-tetrahydro-ß-carbolin
1-Methylen-2-acetyl-7-methoxy-1,2,3,4-tetrahydro-β-carbolin
9-Methoxy-2,3,4,6,7,12-hexahydroindolo[2,3-a]quinolizin-4-on.

9. Kosmetische Zusammensetzung, **dadurch gekennzeichnet, dass** sie als kosmetisch wirksame Verbindung ein Derivat der allgemeinen Formel I 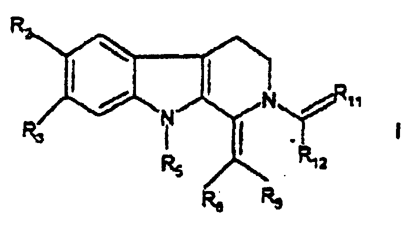 gemäß einem der Ansprüche 1 bis 5 enthält.

10. Verfahren zur Herstellung der Derivate der allgemeinen Formel I 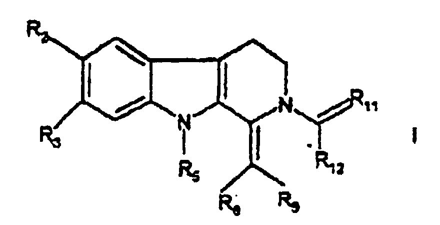 gemäß einem der Ansprüche 1 bis 5, **dadurch gekennzeichnet, dass** man eine der folgenden Reaktionen verwendet:
10.1 Wenn R₁₁ ein Sauerstoffatom darstellt und R₉ und R₁₂ keinen Zyklus bilden, können die Derivate der Formel I direkt erhalten werden, indem die Verbindungen der allgemeinen Formel IIa 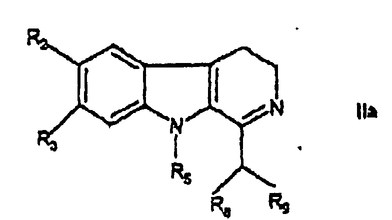 in der R₂, R₃, R₅, R₈ und R₉ wie oben definiert sind, mit einem Acylierungsmittel (Chlorsäure, Säureanhydrid, Chlorformiat, Isocyanat) umgesetzt werden oder durch Austausch mit einem Ester, wodurch die Derivate der Formel I'a erhalten werden, 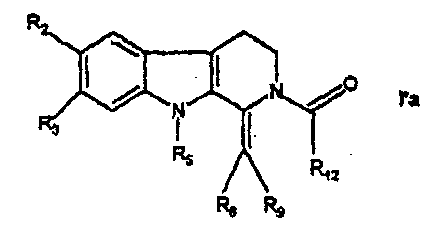 in der R₂, R₃, R₅, R₈, R₉ und R₁₂ wie oben definiert sind;
10.2 Zum Erhalt der Derivate der allgemeinen Formel IIa wird eine Bischler-Napieralski-Reaktion durchgeführt, in der die Verbindungen der allgemeinen Formel IIIa 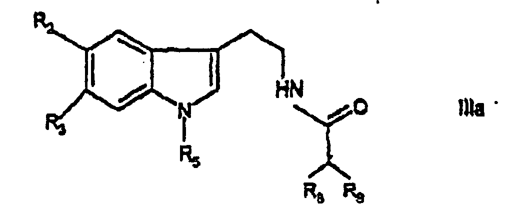 in der R₂, R₃, R₅, R₈, R₉ wie oben definiert sind, mit einem Phosphoranhydrid (P₂O₅) oder Phosphoroxychlorid (POCl₃) in einem geeigneten Lösungsmittel, beispielsweise Toluol oder Xylol, umgesetzt werden;
10.3 Die Derivate der allgemeinen Formel I, in denen R₉ und R₁₂ unter Bildung eines Zyklus verbunden sind, werden erhalten, indem bei einem zyklischen Imid der allgemeinen Formel IIIc 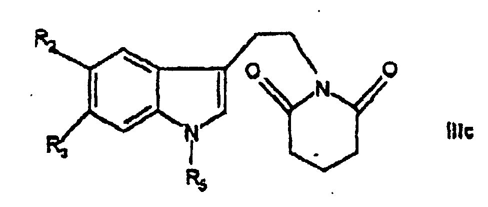 in der R₂, R₃, R₅ wie oben definiert sind, unter den oben beschriebenen Bedingungen eine Bischler-Napieralski-Reaktion durchgeführt wird, wodurch das Derivat der allgemeinen Formel I'c erhalten wird, 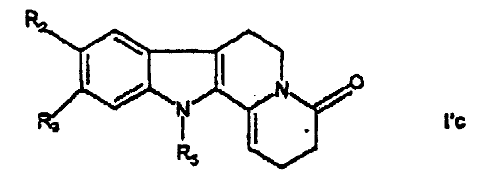 in der R₂, R₃, R₅ wie oben definiert sind.

## Claims

1. Carboline derivatives of general formula I 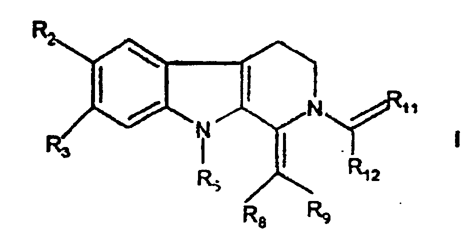 in which
- R₂ and R₃ represent, independently of each other, a hydrogen atom, a hydroxyl radical, a C₁-C₆ alkyl radical, an alkoxy radical whose alkyl residue is C₁-C₆, an aryloxy radical, an aralkoxy radical whose alkyl residue is C₁-C₆, a halo radical, a nitro radical or an unsaturated C₂-C₆ hydrocarbon chain, provided that at least one of the substituents R₂ or R₃ represents a hydroxyl or alkoxy radical whose alkyl residue is C₁-C₆,
- R₅ represents a hydrogen atom or a C₁-C₆ alkyl radical,
- R₈ and R₉ represent, independently of each other, a hydrogen atom, a C₁-C₆ alkyl radical or a phenyl group,
- R₁₂ represents a hydrogen atom, a C₁-C₆ alkyl radical, an aryl radical, an aralkyl radical whose alkyl residue is C₁-C₆, an unsaturated C₂-C₆ hydrocarbon chain, each being optionally substituted with one or more halogens, an amino radical, an alkylamino radical whose alkyl residue is C₁-C₆, a dialkylamino radical whose alkyl residue is C₁-C₆, an arylamino radical, a diarylamino radical, an aralkylamino radical whose alkyl residue is C₁-C₆, or an aryl(alkyl)amino radical whose alkyl residue is C₁-C₆, an arylcarbonyl radical, an alkoxycarbonyl radical whose alkyl residue is C₁-C₆ or an alkoxy radical whose alkyl residue is C₁-C₆,
- or R₉ and R₁₂ are linked to form a ring comprising 5 or 6 atoms, and capable of being substituted with one or more groups representing, independently of each other, a C₁-C₆ alkyl radical,
- R₁₁ represents an oxygen or sulphur atom,
in which the term aryl represents a phenyl, thienyl, furanyl, pyridyl or naphthyl group optionally substituted with one or more substituents chosen from a C₁-C₆ alkyl radical, an alkoxy radical whose alkyl residue is C₁-C₆ or a halo radical,
with the exception of l-methylene-2-acetyl-7-methoxy-1,2,3,4-tetrahydro-β-carboline,
the racemic mixtures thereof, the enantiomers thereof in the pure state or as a mixture, and the therapeutically acceptable salts thereof.

2. Derivatives of general formula I according to Claim 1, **characterized in that** R₁₁ represents an oxygen atom.

3. Derivatives of general formula I according to either of Claims 1 and 2, **characterized in that** at least one of the substituents R₂ or R₃ represents a methoxy radical.

4. Derivatives of general formula I according to one of Claims 1 to 3, **characterized in that** R₁₂ represents a methyl, an ethyl, an n-propyl, an aryl, an aralkyl with a C₁-C₆ alkyl residue, a perfluoromethyl, perfluoroethyl or perfluoropropyl radical or an arylamino radical.

5. Derivatives of general formula I according to Claim 1, **characterized in that** they are selected from the following derivatives:
1-Methylene-2-pentafluoropropionyl-6-methoxy-1,2,3,4-tetrahydro-β-carboline
1-Methylene-2-trifluoroacetyl-6-methoxy-1,2,3,4-tetrahydro-β-carboline
1-Methylene-2-formyl-6-methoxy-1,2,3,4-tetrahydro-β-carboline
1-Methylene-2-acetyl-6-methoxy-1,2,3,4-tetrahydro-β-carboline
1-Methylene-2-propionyl-6-methoxy-1,2,3,4-tetrahydro-β-carboline
1-Methylene-2-butyryl-6-methoxy-1,2,3,4-tetrahydro-β-carboline
1-Methylene-2-benzoyl-6-methoxy-1,2,3,4,tetrahydro-β-carboline
1-Methylene-2-ethoxycarbonyl-6-methoxy-1,2,3,4-tetrahydro-β-carboline
1-Methylene-2-(N-phenyl)carbamoyl-6-methoxy-1,2,3,4-tetrahydro-β-carboline
1-Methylene-2-acetyl-6-methoxy-9-methyl-1,2,3,4-tetrahydro-β-carboline
1-Methylene-2-acetyl-6,7-dimethoxy-1,2,3,4-tetrahydro-β-carboline
9-Methoxy-2,3,4,6,7,12-hexahydroindolo[2,3-a]-quinolizin-4-one.

6. Medicinal product, **characterized in that** it comprises at least one β-carboline derivative of general formula I 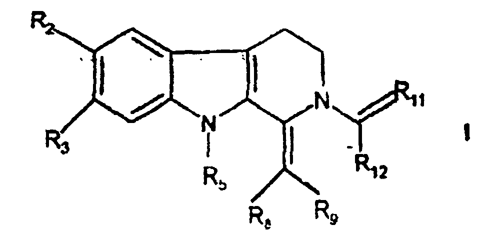 according to one of Claims 1 to 5, or 1-methylene-2-acetyl-7-methoxy-1,2,3,4-tetrahydro-β-carboline.

7. Medicinal product according to Claim 6, **characterized in that** it is a hypnotic medicinal product.

8. Medicinal product according to Claim 6 or 7, **characterized in that** it comprises a derivative selected from:
1-Methylene-2-pentafluoropropionyl-6-methoxy-1,2,3,4-tetrahydro-β-carboline
1-Methylene-2-trifluoroacetyl-6-methoxy-1,2,3,4-tetrahydro-β-carboline
l-Methylene-2-formyl-6-methoxy-1,2,3,4-tetrahydro-β-carboline
1-Methylene-2-acetyl-6-methoxy-1,2,3,4-tetrahydro-β-carboline
1-Methylene-2-propionyl-6-methoxy-1,2,3,4-tetrahydro-β-carboline
1-Methylene-2-butyryl-6-methoxy-1,2,3,4-tetrahydro-β-carboline
1-Methylene-2-benzoyl-6-methoxy-1,2,3,4-tetrahydro-β-carboline
1-Methylene-2-ethoxycarbonyl-6-methoxy-1,2,3,4-tetrahydro-β-carboline
1-Methylene-2-(N-phenyl)carbamoyl-6-methoxy-1,2,3,4-tetrahydro-β-carboline
1-Methylene-2-acetyl-6-methoxy-9-methyl-1,2,3,4-tetrahydro-β-carboline
1-Methylene-2-acetyl-6,7-dimethoxy-1,2,3,4-tetrahydro-β-carboline
1-Methylene-2-acetyl-7-methoxy-1,2,3,4-tetrahydro-β-carboline
9-methoxy-2,3,4,6,7,12-hexahydroindolo[2,3-a]-quinolizin-4-one.

9. Cosmetic composition, **characterized in that** it comprises, as cosmetically active compound, a derivative of general formula I, 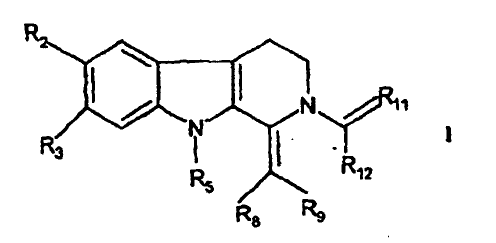 according to one of Claims 1 to 5.

10. Process for the preparation of the derivatives of general formula I, 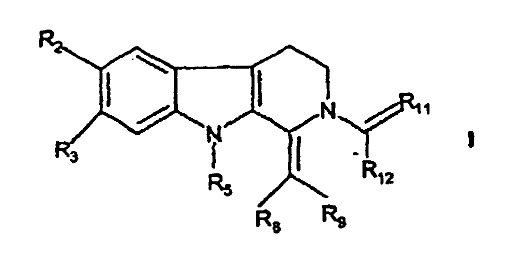 according to Claims 1 to 5, **characterized in that** one of the following reactions is used:
10.1. when R₁₁ represents an oxygen atom, and when R₉ and R₁₂ do not form a ring, the derivatives of formula I may be obtained directly by reacting the compounds of general formula IIa, 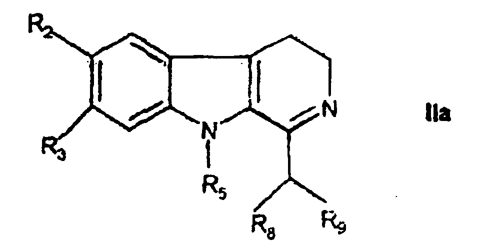 for which R₂, R₃, R₅, R₈, R₉ are defined above, with an acylating agent (acid chloride, acid anhydride, chloroformate, isocyanate) or by exchange with an ester, and the derivatives of formula I'a 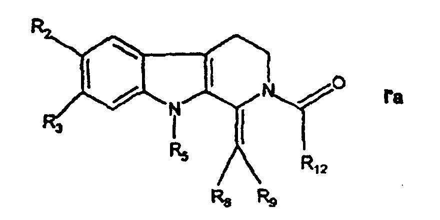 in which R₂, R₃, R₅, R₈, R₉ and R₁₂ are defined above, are thus obtained;
10.2. in order to obtain the derivatives of general formula IIa, a Bischler-Napieralski reaction is carried out by reacting the compounds of general formula IIIa 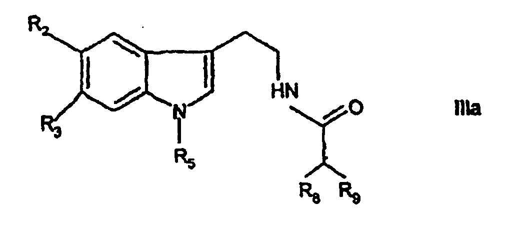 in which R₂, R₃, R₅, R₈, R₉ are defined above, with phosphorus pentoxide (P₂O₅) or phosphorus oxychloride (POCl₃), in a suitable solvent, for example toluene or xylene;
10.3. the derivatives of general formula I, for which R₉ and R₁₂ are linked in order to form a ring, are obtained by carrying out a Bischler-Napieralski reaction, under the conditions described above, on a cyclic imide of general formula IIIc 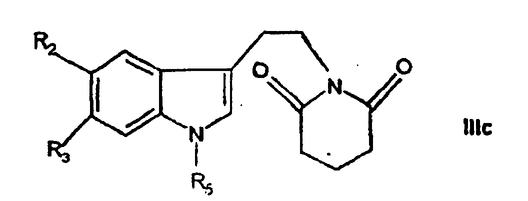 for which R₂, R₃, R₅ are defined above, and the derivative of general formula I'c 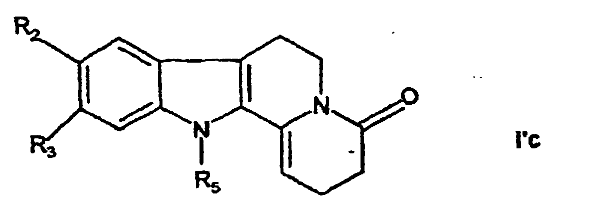 for which R₂, R₃, R₅ are defined above, is thus obtained.
